# EUROPEAN PATENT APPLICATION

(11) **EP 4 303 296 A1**
(43) Date of publication of application: **10.01.2024**
(21) Application number: 22763292.4
(22) Date of filing: 02.03.2022
(51) Int. Cl.: C12M 3/00, C12M 1/00

(54) **SAMPLING DEVICE AND CELL CULTURE SYSTEM**

(30) Priority: 03.03.2021 JP 2021033629
(71) Applicant: TERUMO Kabushiki Kaisha, Tokyo 151-0072 (JP)
(72) Inventor: IGARASHI, Masatsugu, Ashigarakami-gun, Kanagawa 259-0151 (JP)
(74) Representative: TBK
(86) International application number: PCT/JP2022/008731
(87) International publication number: WO 2022/186238

(57) **Abstract**

A cell culturing system (10) includes a culturing device (11) including a plurality of reactors (12), and a sampling device (60) that collects a liquid sample from the culturing device (11). The sampling device (60) includes a temporary storage unit (136) that is provided in a sample introduction channel (130) and can temporarily store a sample. A sample in each of the plurality of reactors (12) sequentially flows into the temporary storage unit (136) under the operation of an upstream pump (142), by which a combined sample obtained by combining a plurality of samples is generated. The temporary storage unit (136) sends the combined sample to the sampling channel (64).

## Description

### Technical Field

The present invention relates to a sampling device for collecting a liquid sample from a culturing device that cultures cells, and a cell culturing system.

### Background Art

For example, U.S. Patent No. 9442047 discloses a sampling device including a sampling channel for collecting a liquid sample from a culturing device. The sampling device includes an introduction pump that draws a sample from a sample introduction channel connected to the culturing device into the sampling channel, and a detection unit provided on a downstream side of the sampling channel. The detection unit detects components contained in the sample and amounts (concentrations) of the components.

### Summary of Invention

This type of culturing device may have a plurality of reactors as culture vessels in order to improve the efficiency of cell culture. That is, the culturing device can culture cells in each of the plurality of reactors by, for example, seeding cells on the reactor and supplying a culture medium to the reactor.

Meanwhile, the plurality of reactors slightly differs in culture states due to factors such as a slight difference in a flowing state of the culture medium. Therefore, even if a system constructed by connecting a sampling device to a culturing device for detecting a sample detects a sample flowing out from an unspecified reactor, the culture state of each reactor cannot be accurately grasped.

On the other hand, if the sampling device is configured to detect a sample for each of the plurality of reactors, the number of times of sampling and the sample amount greatly increase, which may decrease the sampling efficiency.

The present invention has been made in view of the above circumstances, and an object thereof is to provide a sampling device and a cell culturing system capable of more efficiently detecting a sample for each of a plurality of reactors.

In order to achieve the above object, a first aspect of the present invention provides a sampling device for collecting a sample in a liquid form from a culturing device that has a plurality of reactors for culturing a cell on the basis of a flow of a culture medium, the sampling device including: a sampling channel through which the sample flows; a detection unit provided in the sampling channel; a sample introduction channel that connects the sampling channel on an upstream side with respect to the detection unit and the culturing device; a pump that allows the sample to flow through the sample introduction channel; and a control unit that operates the pump, the sampling device including a temporary storage unit provided in the sample introduction channel and capable of temporarily storing the sample, wherein the temporary storage unit sequentially receives flow of the sample for each of the plurality of reactors under the operation of the pump to store a combined sample obtained by combining a plurality of the samples, and sends the combined sample to the sampling channel.

In addition, in order to achieve the above object, a second aspect of the present invention provides a cell culturing system for collecting a sample in a liquid form from a culturing unit that has a plurality of reactors for culturing a cell on the basis of a flow of a culture medium, the cell culturing unit sequentially supplying the culture medium to the plurality of reactors, the cell culturing system including: a sampling channel through which the sample flows; a detection unit provided in the sampling channel; a sample introduction channel that connects the sampling channel on an upstream side with respect to the detection unit and the culturing unit; a pump that allows the sample to flow through the sample introduction channel; and a control unit that operates the pump, the cell culturing system including a temporary storage unit provided in the sample introduction channel and capable of temporarily storing the sample, wherein the temporary storage unit sequentially receives flow of the sample for each of the plurality of reactors under the operation of the pump to store a combined sample obtained by combining a plurality of the samples, and sends the combined sample to the sampling channel.

The sampling device and the cell culturing system can more efficiently detect a sample for each of a plurality of reactors.

### Brief Description of Drawings

Fig. 1 is a perspective view schematically illustrating an overall configuration of a cell culturing system to which a sampling device according to an embodiment of the present invention is applied.
Fig. 2 is an explanatory diagram schematically illustrating a channel for a culture medium during cell culture.
Fig. 3 is an explanatory diagram schematically illustrating a channel through which a sample flows out from a plurality of reactors.
Fig. 4 is an explanatory diagram schematically illustrating a channel of the sampling device.
Fig. 5 is a flowchart illustrating a sampling method performed by the sampling device.
Fig. 6 is a flowchart illustrating a sampling step.
Fig. 7 is an explanatory diagram illustrating an operation in a temporary storage step.
Fig. 8 is an explanatory diagram illustrating an operation in a combined sample outflow step.
Fig. 9 is an explanatory diagram schematically illustrating a channel of a sampling device according to a first modification.
Fig. 10A is a flowchart illustrating a sampling step performed by the sampling device according to the first modification. Fig. 10B is a flowchart illustrating a culturing step performed by the culturing device.
Fig. 11 is an explanatory diagram schematically illustrating a channel of a sampling device according to a second modification.

### Description of Embodiments

Preferred embodiments of the present invention will now be described in detail with reference to the drawings.

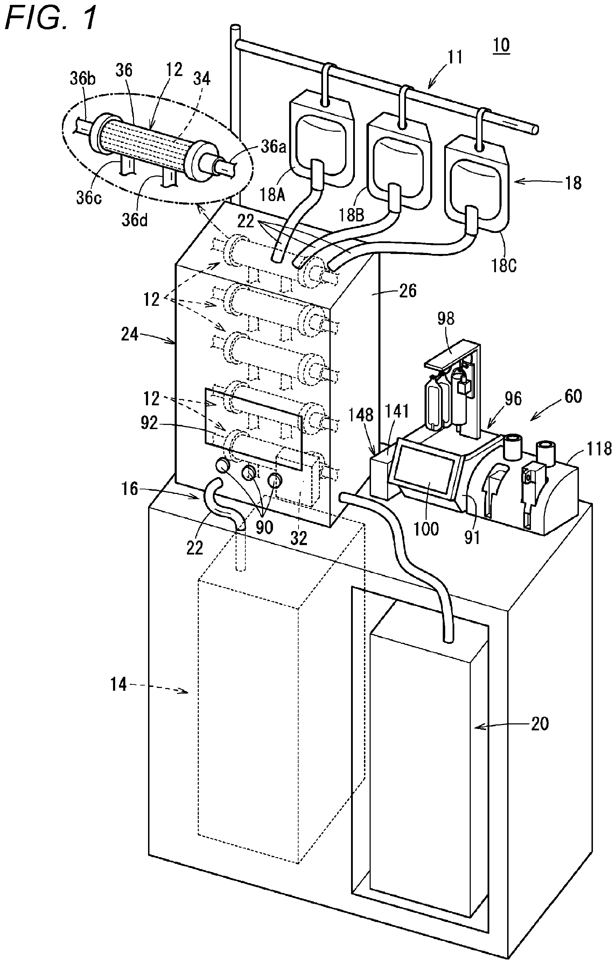

A sampling device 60 according to an embodiment of the present invention is applied to a cell culturing system 10 for culturing biological cells in regenerative medicine as illustrated in Fig. 1. The sampling device 60 samples a culture medium during culture of the cells by the cell culturing system 10, and measures the state of the culture medium. For example, the cell culturing system 10 continues cell culture for a long period of time by discharging lactic acid, carbon dioxide, or the like (including unused medium and oxygen) generated during cell culture from a reactor 12 which is a cell culture vessel while supplying a culture medium or oxygen to the reactor 12.

The biological cells are not particularly limited, and examples thereof include cells contained in blood (T cells and the like) and stem cells (ES cells, iPS cells, mesenchymal stem cells, etc.). As the culture medium, an appropriate culture medium may be selected according to biological cells, and examples thereof include those prepared by adding various amino acids, vitamins, serum, and the like to a basic solution that is, for example, a balanced salt solution (BSS).

The cell culturing system 10 includes a culturing device 11 (culturing unit) in which multiple reactors 12 are set and cells are actually cultured, and a sampling device 60 (sampling unit) that collects a liquid sample from the culturing device 11 during culture. That is, the cell culturing system 10 is configured such that a culture medium flows through and is cultured in each of the multiple reactors 12, whereby several times as many as cells cultured in one reactor 12 can be obtained without greatly changing the culturing period. Although Fig. 1 illustrates the culturing device 11 including five reactors 12, it is obvious that the number of reactors 12 provided in the culturing device 11 is not particularly limited. In addition, the cell culturing system 10 may have a configuration in which a plurality of culturing devices 11 is connected to one sampling device 60. Although the present embodiment has described the cell culturing system 10 in which the culturing unit and a sampling unit are separately provided, the cell culturing system 10 may be a device in which the culturing unit and the sampling unit are integrated (unified).

The culturing device 11 includes a culture medium reservoir 14 storing a culture medium, a flow channel 16 provided between the reactor 12 and the culture medium reservoir 14, a plurality of medical bags 18 connected to the flow channel 16, and a waste liquid unit 20 storing a liquid discharged through the flow channel 16.

A hard tank capable of storing a large amount of culture medium is applied to the culture medium reservoir 14. The flow channel 16 is constituted by multiple tubes 22, and the multiple tubes 22 are respectively connected to the multiple reactors 12, the culture medium reservoir 14, the plurality of medical bags 18, and the waste liquid unit 20.

Examples of the plurality of medical bags 18 include a cell solution bag 18A storing a liquid (cell solution) containing cells, a cleaning solution bag 18B storing a cleaning solution, a stripping solution bag 18C storing a stripping solution, and a collection bag (not illustrated) for collecting cultured cells. The cleaning solution is a liquid used at the time of priming of the reactor 12 and the flow channel 16. Examples of the cleaning solution include a buffer solution such as phosphate buffered salts (PBS) and tris-buffered saline (TBS), and a physiological saline solution. The stripping solution is a liquid for stripping the cells cultured by a culture treatment. As the stripping solution, trypsin or EDTA solution can be applied, for example.

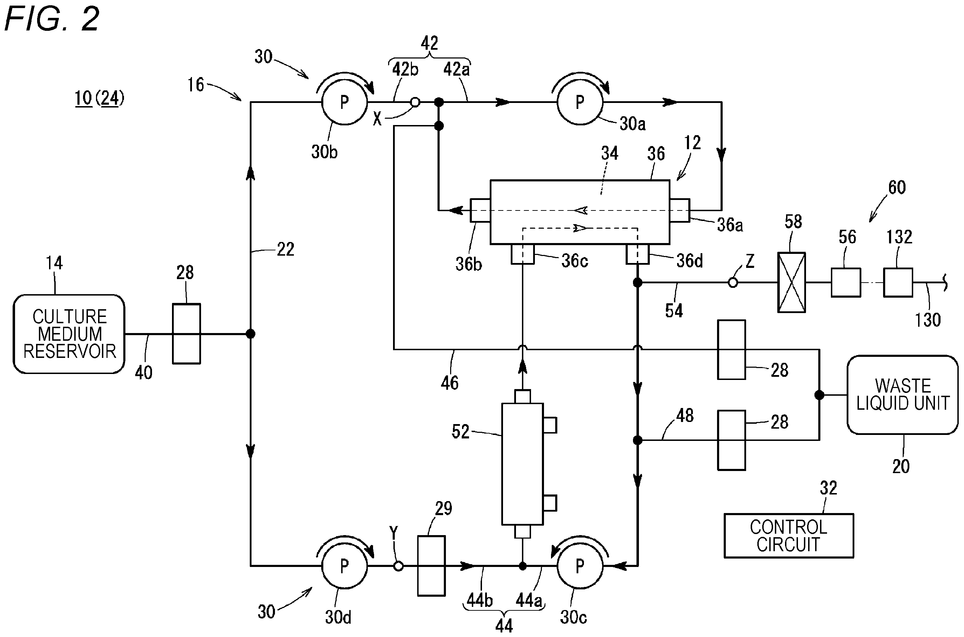

When the cell culturing system 10 is constructed, the flow channel 16 is set so as to pass through a flow path control mechanism 24 of the culturing device 11. The flow path control mechanism 24 includes a housing 26 that houses a part of the flow channel 16. The flow path control mechanism 24 also includes, in the housing 26, a clamp 28 that opens and closes a predetermined tube 22, a pump 30 that allows a liquid in the tube 22 to flow, and a control circuit 32 that controls operations of the clamp 28 and the pump 30 (see Fig. 2).

The multiple reactors 12 are accommodated in the housing 26 of the flow path control mechanism 24. The reactor 12 includes a plurality of (10,000 or more, for example) hollow fibers 34 and a case 36 that accommodates the plurality of hollow fibers 34. Each of the hollow fibers 34 has a lumen (not illustrated), and cells are seeded on an inner peripheral surface defining the lumen. In addition, each of the hollow fibers 34 has a plurality of pores (not illustrated) that allows communication between the outside and the lumen, and each pore transmits a solution or a low-molecular-weight substance without transmitting cells or proteins. A culture medium or the like is supplied to the cells seeded on the inner peripheral surface of the hollow fiber 34 through the lumen or the pores. Hereinafter, the configuration in which the liquid mainly flows through the lumen of the hollow fiber 34 is also referred to as intra capillary (IC), and the configuration in which the liquid mainly flows through the outer side of the hollow fiber 34 is also referred to as extra capillary (EC).

Each of the cases 36 includes a first IC terminal 36a and a second IC terminal 36b which communicate with the lumens of the hollow fibers 34, and a first EC terminal 36c and a second EC terminal 36d which communicate with a space outside the hollow fibers 34 in the case 36. The tube 22 is connected to each terminal.

The configurations of the flow channel 16 between one reactor 12 and the culture medium reservoir 14 and the flow path control mechanism 24 will be specifically described below with reference to Fig. 2. The flow channel 16 includes a medium delivery route 40 connected to the culture medium reservoir 14, and an IC route 42 (internal route) and an EC route 44 (external route) which are branched from the medium delivery route 40. The IC route 42 is a channel for supplying a liquid to the lumen of the hollow fibers 34. The EC route 44 is a path for supplying liquid into the case 36 outside the hollow fibers 34.

The IC route 42 includes an IC circulation circuit 42a capable of circulating liquid with the reactor 12, and an IC supply circuit 42b through which liquid can flow from the culture medium delivery route 40 to the IC circulation circuit 42a. The IC circulation circuit 42a is connected to the first IC terminal 36a and the second IC terminal 36b of the reactor 12, and includes an IC circulation pump 30a that allows liquid to flow through the lumen of the hollow fibers 34. An IC waste liquid circuit 46 that discharges a culture medium to the waste liquid unit 20 is connected to the IC circulation circuit 42a on the downstream side of the reactor 12. On the other hand, the IC supply circuit 42b is provided with an IC supply pump 30b for allowing liquid to flow from the culture medium delivery route 40 to the IC circulation circuit 42a.

On the other hand, the EC route 44 includes an EC circulation circuit 44a capable of circulating liquid with the reactor 12, and an EC supply circuit 44b through which liquid can flow from the culture medium delivery route 40 to the EC circulation circuit 44a. The EC circulation circuit 44a is connected to the first EC terminal 36c and the second EC terminal 36d of the reactor 12, and includes an EC circulation pump 30c that circulates liquid on the outside of the hollow fibers 34. A gas exchanger 52 is provided upstream of the reactor 12 in the EC circulation circuit 44a. The gas exchanger 52 discharges carbon dioxide mixed in the culture medium, and mixes a predetermined gas component (for example, nitrogen N₂: 75%, oxygen O₂: 20%, carbon dioxide CO₂: 5%) with the culture medium. An EC waste liquid circuit 48 that discharges a culture medium to the waste liquid unit 20 is connected to the EC circulation circuit 44a on the downstream side of the reactor 12. The EC supply circuit 44b is provided with an EC supply pump 30d for allowing liquid to flow from the culture medium delivery route 40 to the EC circulation circuit 44a.

Although not illustrated, a plurality of medical bags 18 (cell solution bag 18A, cleaning solution bag 18B, and stripping solution bag 18C) is connected to the IC supply circuit 42b on the upstream side of the IC supply pump 30b or the EC supply circuit 44b on the upstream side of the EC supply pump 30d via a plurality of tubes 22 in addition to the culture medium reservoir 14. Note that these medical bags 18 may be replaced with a collection bag or the like using a sterile connecting device that sterilizes and bonds the bag depending on the intended use.

Then, the sampling device 60 is connected to the EC circulation circuit 44a of the culturing device 11 at a position (between the reactor 12 and the EC waste liquid circuit 48) near the downstream side (second EC terminal 36d) of the reactor 12. Therefore, one end of a sample outflow channel 54 through which a culture medium as a liquid sample flows out is connected to the EC circulation circuit 44a. A culturing-device-side connector 56 is provided at the other end of the sample outflow channel 54. The culturing-device-side connector 56 is mutually connectable to a sampling-device-side connector 132 of the sampling device 60. Note that the sampling device 60 may be connected to the IC circulation circuit 42a on the downstream side (second IC terminal 36b) of the reactor 12 via the sample outflow channel 54.

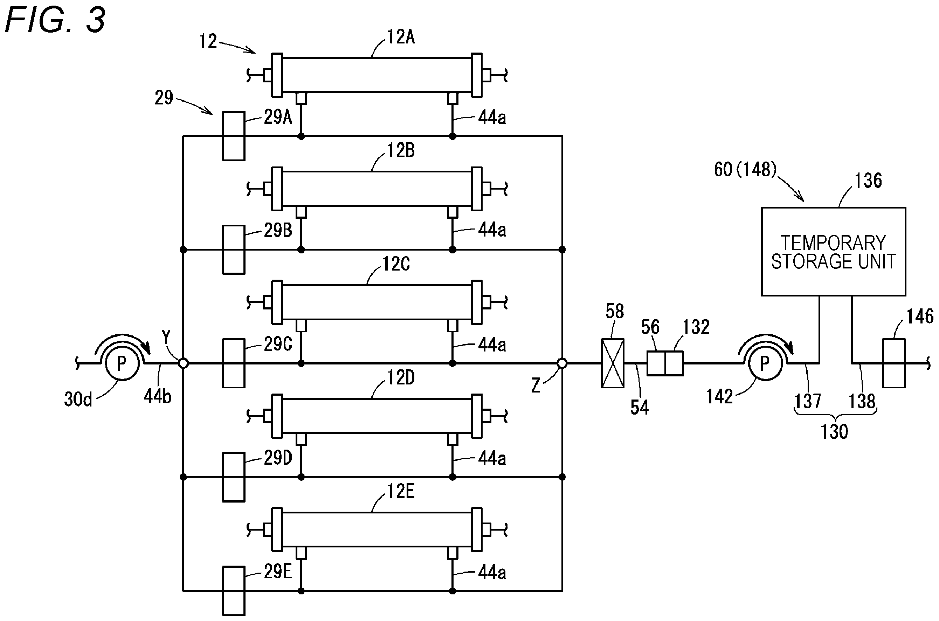

As illustrated in Figs. 2 and 3, the cell culturing system 10 includes multiple IC circulation circuits 42a and multiple EC circulation circuits 44a corresponding to the multiple (five) reactors 12. That is, another IC circulation circuit 42a and another EC circulation circuit 44a (not illustrated) that circulate liquid to another reactor 12 are connected in parallel to a branch point X between the IC supply pump 30b and the IC circulation circuit 42a and a branch point Y between the EC supply pump 30d and the EC circulation circuit 44a. The EC supply circuit 44b between the branch point Y and each EC circulation circuit 44a is provided with a supply clamp 29 for switching between supply and stop of the supply of the culture medium to the corresponding EC circulation circuit 44a.

The five reactors 12 in Fig. 3 are referred to as reactors 12A to 12E below in order from the top to the bottom. The supply clamps 29 provided in the EC supply circuits 44b are referred to as supply clamps 29A to 29E corresponding to the reactors 12A to 12E, respectively. The culturing device 11 opens any one of the supply clamps 29A to 29E and closes the other four clamps while rotating the EC supply pump 30d. As a result, the culture medium is supplied to the EC circulation circuit 44a in which the supply clamp 29 is opened, and the culture medium flows through the reactor 12 along with the circulation in the EC circulation circuit 44a. Although not illustrated, the culturing device 11 includes multiple IC circulation circuits 42a corresponding to the reactors 12A to 12E. The IC supply circuit 42b connected to each of the multiple IC circulation circuits 42a is also provided with a supply clamp (not illustrated) for selectively allowing the culture medium to flow.

In order to be connected to each of the multiple reactors 12, the sample outflow channel 54 branches at a branch point Z and is connected to each EC circulation circuit 44a. An aseptic filter 58 is provided in the sample outflow channel 54 on the culturing-device-side connector 56 side with respect to the branch point Z. The aseptic filter 58 maintains an aseptic state of the culture medium circulating through the culturing device 11 (EC circulation circuit 44a).

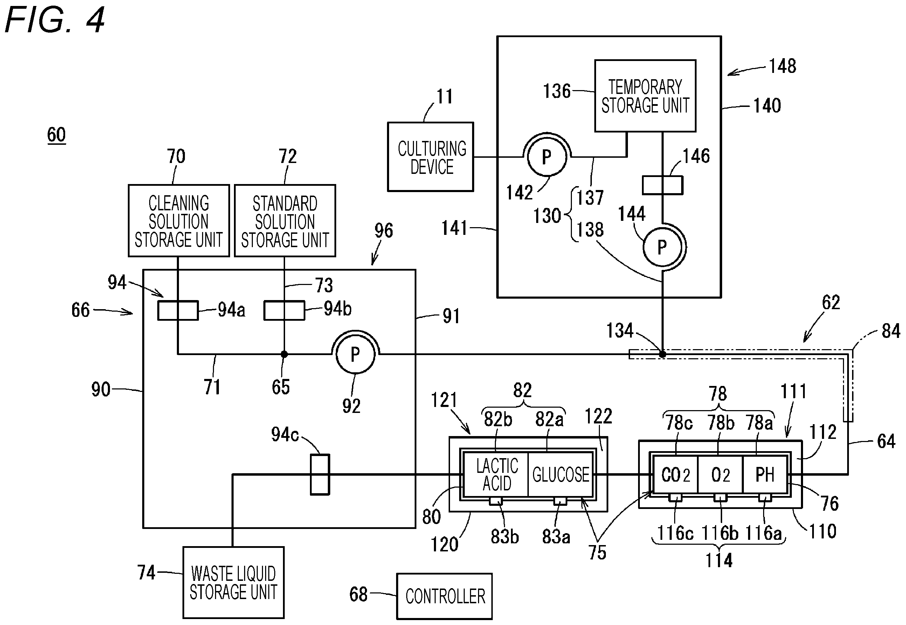

Next, a configuration of the sampling device 60 will be described with reference to Fig. 4. The sampling device 60 collects a sample of a culture medium from one or more culturing devices 11, and detects components contained in the sample and amounts (concentrations) of the components. The sampling device 60 includes a sampling kit 62 having a sampling channel 64 through which a sample is collected, a plurality of mechanisms 66 in which the sampling kit 62 is detachably set, and a controller 68 that controls operations of the plurality of mechanisms 66. The sampling kit 62 is a disposable product that is used and thrown away, and the plurality of mechanisms 66 is a reusable product that can be reused.

In addition to the sampling channel 64, the sampling kit 62 includes a cleaning solution storage unit 70, a standard solution storage unit 72, a waste liquid storage unit 74, and a detection unit 75 (first detection unit 76 and second detection unit 80). The sampling channel 64 is constituted by a flexible tube having an appropriate thickness by which the sample can pass therethrough. The cleaning solution storage unit 70 is connected to a branch point 65 to which one end of the sampling channel 64 is connected via a cleaning solution branch path 71, and the standard solution storage unit 72 is connected to the branch point 65 via a standard solution branch path 73. The other end of the sampling channel 64 is connected to the waste liquid storage unit 74.

The cleaning solution storage unit 70 and the standard solution storage unit 72 are made of, for example, a soft resin material such as polyvinyl chloride or polyolefin, and formed into a bag shape (medical bag). The cleaning solution storage unit 70 and the standard solution storage unit 72 are not particularly limited as long as they can store liquid. The waste liquid storage unit 74 shares a tank of the waste liquid unit 20 of the culturing device 11, but it is not limited thereto, and a medical bag or the like may be applied.

The cleaning solution storage unit 70 stores a cleaning solution. The cleaning solution is not particularly limited, and for example, a buffer solution, a physiological saline solution, or the like described as the cleaning solution in the cleaning solution bag 18B of the culturing device 11 may be appropriately employed.

The standard solution storage unit 72 stores a standard solution. The standard solution is a liquid for calibrating the first detection unit 76 and the second detection unit 80, and has a PH value, a glucose value (glucose concentration), and a lactic acid value (lactic acid concentration) which are set to prescribed values.

The first detection unit 76 and the second detection unit 80 are provided in series and separated from each other at an intermediate position of the sampling channel 64. Note that the detection unit 75 is not limited to be divided into the first detection unit 76 and the second detection unit 80, and may have a structure in which the first detection unit 76 and the second detection unit 80 are integrated, or a structure divided into three or more units.

The first detection unit 76 is a tubular member having multiple first elements 78 that come in contact with (in liquid contact with) the sample in a flow path in the sampling channel 64. Examples of the multiple first elements 78 include a PH chip 78a for measuring the PH in the sample, an O₂ chip 78b for measuring the O₂ concentration in the sample, and a CO₂ chip 78c for measuring the CO₂ concentration in the sample. The PH chip 78a is colored by reaction with H⁺ and OH-. The O₂ chip 78b is colored by reaction with O₂. The CO₂ chip 78c is colored by reaction with CO₂.

The second detection unit 80 is a tubular member having multiple second elements 82 that come in contact with (in liquid contact with) the sample in the flow path in the sampling channel 64, and is provided on the downstream side (waste liquid storage unit 74 side) with respect to the first detection unit 76. For example, the multiple second elements 82 are biosensors that react an enzyme with a circulating sample and detect a current change or the like. Examples of the multiple second elements 82 include a glucose chip 82a that measures the glucose concentration in the sample and a lactic acid chip 82b that measures the lactic acid concentration in the sample. The glucose chip 82a is electrically connected to a glucose terminal 83a protruding to the outside of the tubular member. The lactic acid chip 82b is electrically connected to a lactic acid terminal 83b protruding to the outside of the tubular member.

In addition, the sampling kit 62 includes a connection part 84 to which one or more sample introduction channels 130 described later can be connected between the branch point 65 of the sampling channel 64 and the first detection unit 76. The connection part 84 is, for example, a member obtained by integrally molding a plurality of branch ports each having a valve (not illustrated) that closes when the sample introduction channel 130 is not attached and opens as the sample introduction channel 130 is attached (in Fig. 4, the connection part 84 is indicated as a range surrounded by a two-dot chain line for convenience). Alternatively, a port to which the sample introduction channel 130 can be connected with the sampling channel 64 being kept aseptic can be applied to the connection part 84.

A part of the sampling kit 62 described above is set in a main mechanism 90 which is one of the plurality of mechanisms 66 as illustrated in Fig. 4. The main mechanism 90 also includes, in the housing 91, a main-mechanism-side pump 92 and a plurality of clamps 94 that opens and closes flow paths in the respective channels (tubes). Although not illustrated, it is preferable that the controller 68 that controls the sampling device 60 is also provided in the main mechanism 90. The sampling kit 62 is set in the main mechanism 90, by which a main unit 96 of the sampling device 60 is constructed.

The sampling channel 64 extending between the branch point 65 and the connection part 84 is disposed in the main-mechanism-side pump 92. The main-mechanism-side pump 92 has a circular wound portion around which the sampling channel 64 can be wound so as to wrap around, and rotates so as to apply a peristaltic action on the wrapping sampling channel 64 (tube), thereby allowing an internal fluid (liquid, air, etc.) to flow.

The multiple clamps 94 include a cleaning solution clamp 94a for opening and closing the cleaning solution branch path 71, a standard solution clamp 94b for opening and closing the standard solution branch path 73, and a waste liquid clamp 94c for opening and closing the sampling channel 64 between the second detection unit 80 and the waste liquid storage unit 74.

The first detection unit 76 of the sampling kit 62 is set in a first measuring instrument 110 which is one of the plurality of mechanisms 66, whereby a first sensor unit 111 is constructed. The first measuring instrument 110 includes a holder 112 that accommodates the first detection unit 76, and a measurement body 114 fixed to the holder 112 and optically measuring the plurality of first elements 78.

The measurement body 114 is provided with a PH detector 116a, a O₂ detector 116b, and a CO₂ detector 116c that are arranged to respectively face the PH chip 78a, the O₂ chip 78b, and the CO₂ chip 78c with the first detection unit 76 being held by the holder 112. Under the control of the controller 68, the measurement body 114 emits measurement light having a wavelength corresponding to the characteristics of each of the first elements 78, receives excitation light generated from the first element 78 by excitation, and transmits a detection signal thereof to the controller 68.

Further, the second detection unit 80 of the sampling kit 62 is set in a second measuring instrument 120 which is one of the plurality of mechanisms 66, whereby a second sensor unit 121 is constructed. The second measuring instrument 120 includes a case 122 that can accommodate the second detection unit 80, and an enzyme detector (not illustrated) electrically connected to the glucose terminal 83a and the lactic acid terminal 83b. The enzyme detector detects a current value from each of the glucose chip 82a and the lactic acid chip 82b, and transmits a detection signal based on the current value to the controller 68.

Then, in order to introduce a sample to be measured by the first sensor unit 111 and the second sensor unit 121, the sample introduction channel 130 is connected to the connection part 84 of the sampling kit 62 (sampling channel 64). Similar to the sampling channel 64, the sample introduction channel 130 is constituted by a flexible tube having an appropriate thickness by which the sample can pass therethrough.

The sample introduction channel 130 has, at one end, the sampling-device-side connector 132 to be connected to the culturing-device-side connector 56 (see also Figs. 2 and 3). A plug (not illustrated) attachable to and detachable from the connection part 84 is provided at the other end of the sample introduction channel 130. Hereinafter, a portion where the sample introduction channel 130 is connected to the sampling channel 64 is referred to as a connection point 134.

A temporary storage unit 136 is provided in the sample introduction channel 130 between the sampling-device-side connector 132 and the plug (connection point 134). The temporary storage unit 136 temporarily stores the sample flowing out of the culturing device 11, and then, sends the sample to the sampling channel 64. For example, the temporary storage unit 136 is constituted by a medical bag softer than the sample introduction channel 130, and is hung on the stand 98 fixed on the housing 91 of the main unit 96. The temporary storage unit 136 may be constituted by a hard container.

The sample introduction channel 130 includes an upstream line 137 provided between the sampling-device-side connector 132 and the temporary storage unit 136, and a downstream line 138 provided between the temporary storage unit 136 and the plug. In a state where the temporary storage unit 136 is hung on the stand 98, the upstream line 137 and the downstream line 138 are connected to the lower side of the temporary storage unit 136 in the direction of gravity.

A part of the sample introduction channel 130 is set in the introduction mechanism 140 which is one of the plurality of mechanisms 66, by which an introduction unit 148 of the sampling device 60 is constructed. The introduction mechanism 140 includes an upstream pump 142, an introduction pump 144, and a downstream clamp 146 in a housing 141. The introduction mechanism 140 may also include a sensor (not illustrated) that detects pressure and air bubbles in the flow path of the sample introduction channel 130.

The introduction unit 148 is configured such that a part of the sample introduction channel 130, the upstream pump 142, the introduction pump 144, and the downstream clamp 146 can be integrally handled. The sample introduction channel 130 (downstream line 138) shortly extending from the introduction unit 148 is connected to the connection part 84 on the housing 91.

The upstream pump 142 is disposed on the upstream line 137 (that is, between the culturing device 11 and the temporary storage unit 136) in the introduction unit 148. The introduction pump 144 and the downstream clamp 146 are disposed on the downstream line 138 (that is, between the sampling channel 64 and the temporary storage unit 136) in the introduction unit 148. Each of the upstream pump 142 and the introduction pump 144 has a circular wound portion around which the sample introduction channel 130 can be wound so as to wrap around, and rotates so as to apply a peristaltic action on the wrapping sample introduction channel 130 (tube), thereby allowing an internal fluid to flow. The downstream clamp 146 switches between allowing the sample to flow to the sampling channel 64 from the temporary storage unit 136 and interrupting the flow by opening and closing the downstream line 138.

The controller 68 (control unit) is a computer including one or more processors (not illustrated), a memory, an input/output interface, and an electronic circuit. The controller 68 controls the entire sampling device 60 by the processor executing the program stored in the memory. In the present embodiment, the controller 68 is configured to be able to communicate information with the control circuit 32 of the culturing device 11, and performs ganged control of the culturing device 11 and the sampling device 60. The controller 68 may be a control device integrated with the control circuit 32 of the culturing device 11.

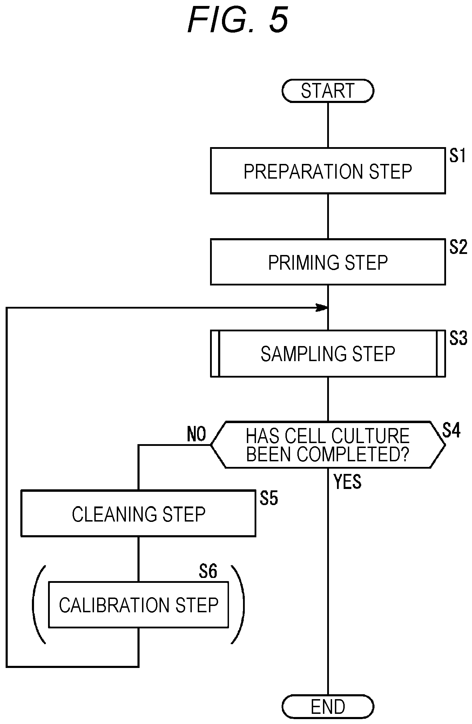

The sampling device 60 according to the present embodiment is basically configured as described above, and a sampling method performed by the sampling device 60 will be described below with reference to Fig. 5. The sampling method includes a preparation step, a priming step, a sampling step, a cleaning step, and a calibration step which are sequentially performed.

First, in the preparation step (step S1), the user of the cell culturing system 10 sets (attaches) the sampling kit 62 to the main mechanism 90 to form the main unit 96 as illustrated in Fig. 4. Thereafter, the user sets the first detection unit 76 exposed from the housing 91 in the first measuring instrument 110 to construct the first sensor unit 111, and sets the second detection unit 80 which are similarly exposed in the second measuring instrument 120 to construct the second sensor unit 121. The first sensor unit 111 and the second sensor unit 121 are suspended from the stand 98.

The user also sets the sample introduction channel 130 in the introduction mechanism 140 to form the introduction unit 148. Thereafter, the user connects the sampling-device-side connector 132 of the sample introduction channel 130 exposed from the introduction unit 148 to the culturing-device-side connector 56, and connects the plug of the sample introduction channel 130 to the connection part 84.

Subsequently, in the priming step (step S2 in Fig. 5), the controller 68 opens the cleaning solution clamp 94a and the waste liquid clamp 94c, closes the standard solution clamp 94b, and rotates the main-mechanism-side pump 92. Thus, the cleaning solution in the cleaning solution storage unit 70 passes through the first detection unit 76 and the second detection unit 80 and is discharged to the waste liquid storage unit 74.

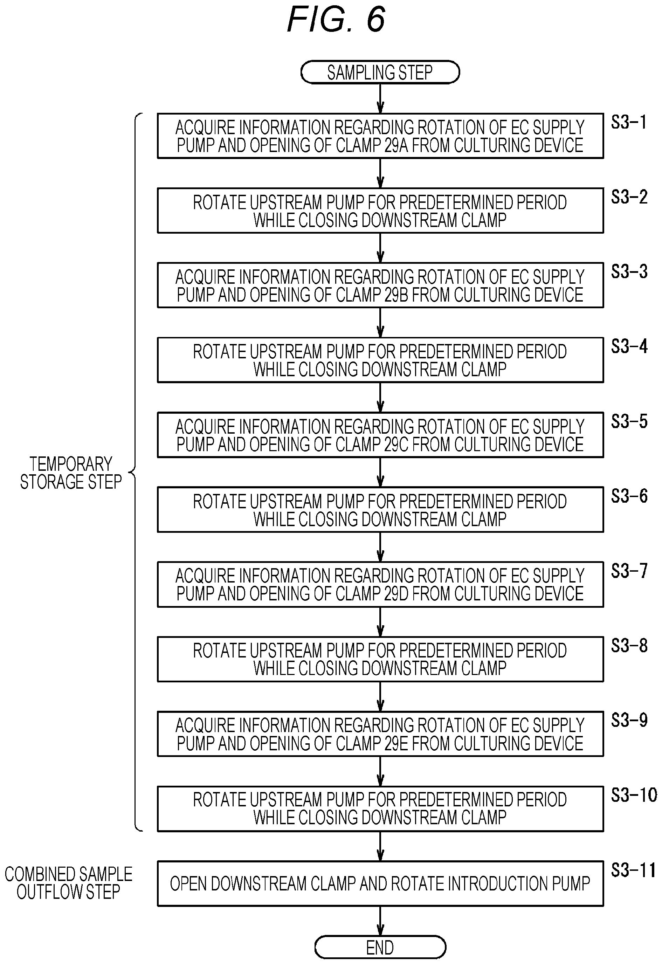

Next, the controller 68 introduces the sample to the detection unit 75 from the culturing device 11 in the sampling step (step S3 in Fig. 5). At this time, the controller 68 sequentially performs a temporary storage step and a combined sample outflow step as illustrated in Fig. 6.

The temporary storage step is for collectively storing the samples flowing out from the reactors 12A to 12E in the temporary storage unit 136. In the present embodiment, the controller 68 acquires information regarding the rotation of the EC supply pump 30d of the culturing device 11 and the opening of each of the supply clamps 29A to 29E, and rotates the upstream pump 142 while each of the supply clamps 29A to 29E is opened. The upstream pump 142 operates at the same rotation speed and for the same time (predetermined period) during opening of each of the supply clamps 29A to 29E. As a result, the samples from the reactors 12A to 12E are stored in the same amount in the temporary storage unit 136.

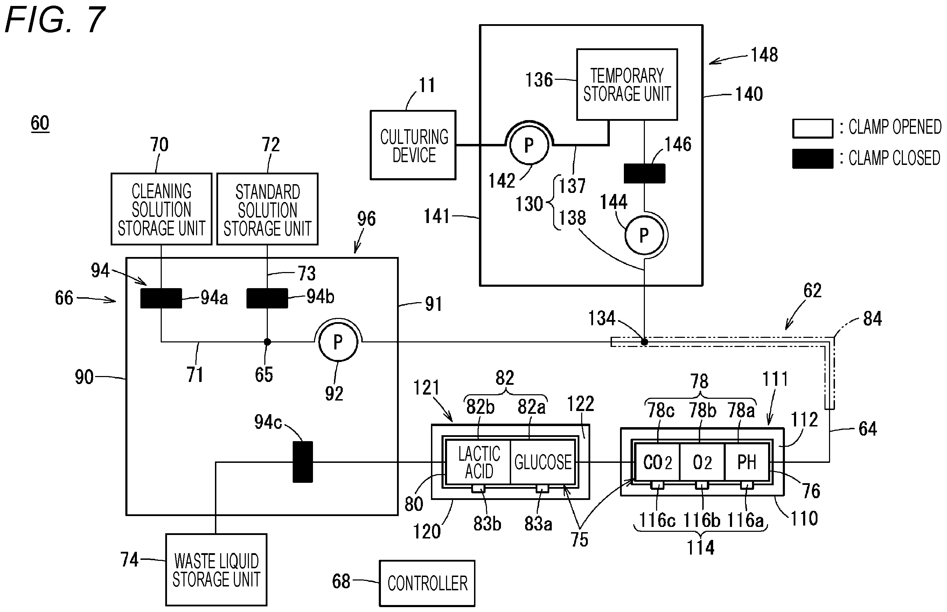

Specifically, after the start of the temporary storage step, the controller 68 acquires information regarding the rotation of the EC supply pump 30d and the opening of the supply clamp 29A from the control circuit 32 of the culturing device 11 (step S3-1). Thereafter, the controller 68 rotates the upstream pump 142 for a predetermined period while closing the downstream clamp 146 (step S3-2). At this time, the controller 68 stops the operation of the introduction pump 144 (the same applies below until the end of the temporary storage step). Thus, the sample flowing out of the reactor 12A is stored in the temporary storage unit 136 via the upstream line 137 as illustrated in Fig. 7.

The controller 68 rotates the upstream pump 142 to allow the sample to flow at a flow rate of 10 mL/min, for example. The predetermined period during which the upstream pump 142 is operated is set to about 5 seconds to 15 seconds although it depends on the opening period of the supply clamp 29A. As a result, when, for example, the upstream pump 142 is rotated for 6 seconds, 1 mL of the sample from the reactor 12A is stored in the temporary storage unit 136.

Next, the controller 68 acquires information regarding the rotation of the EC supply pump 30d and the opening of the supply clamp 29B from the control circuit 32 of the culturing device 11 (step S3-3). Thereafter, the controller 68 rotates the upstream pump 142 for a predetermined period (same as the period for acquiring the sample from the reactor 12A) while closing the downstream clamp 146 (step S3-4). Thus, the sample from the reactor 12B is stored in the temporary storage unit 136 in the same amount as the storage amount of the sample from the reactor 12A.

Next, the controller 68 acquires information regarding the rotation of the EC supply pump 30d and the opening of the supply clamp 29C from the control circuit 32 of the culturing device 11 (step S3-5). Thereafter, the controller 68 rotates the upstream pump 142 for a predetermined period (same as the period for acquiring the sample from the reactor 12A) while closing the downstream clamp 146 (step S3-6). Thus, the sample from the reactor 12C is stored in the temporary storage unit 136 in the same amount as the storage amount of the sample from the reactor 12A.

Next, the controller 68 acquires information regarding the rotation of the EC supply pump 30d and the opening of the supply clamp 29D from the control circuit 32 of the culturing device 11 (step S3-7). Thereafter, the controller 68 rotates the upstream pump 142 for a predetermined period (same as the period for acquiring the sample from the reactor 12A) while closing the downstream clamp 146 (step S3-8). Thus, the sample from the reactor 12D is stored in the temporary storage unit 136 in the same amount as the storage amount of the sample from the reactor 12A.

Next, the controller 68 acquires information regarding the rotation of the EC supply pump 30d and the opening of the supply clamp 29E from the control circuit 32 of the culturing device 11 (step S3-9). Thereafter, the controller 68 rotates the upstream pump 142 for a predetermined period (same as the period for acquiring the sample from the reactor 12A) while closing the downstream clamp 146 (step S3-10). Thus, the sample from the reactor 12E is stored in the temporary storage unit 136 in the same amount as the storage amount of the sample from the reactor 12A.

By performing the above steps S3-1 to S3-10, a combined sample obtained by combining the samples from the reactors 12A to 12E is stored in the temporary storage unit 136. The combined sample is obtained by combining the same amount of the samples from the reactors 12A to 12E. Therefore, in the combined sample, the samples in one culturing device 11 are averaged, and thus, the combined sample is considered to indicate the culture state of the culturing device 11.

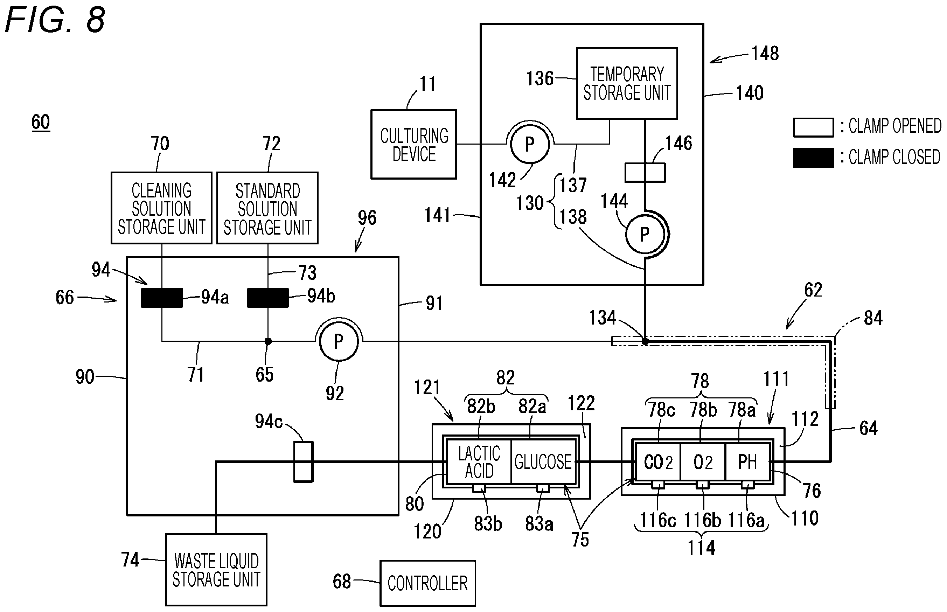

After the temporary storage step, the sampling device 60 supplies the combined sample in the temporary storage unit 136 to the sampling channel 64 in the next combined sample outflow step. During this process, the controller 68 opens the downstream clamp 146 and rotates the introduction pump 144 (step S3-11). During this process, the controller 68 also stops the operation of the upstream pump 142. The controller 68 closes the cleaning solution clamp 94a and the standard solution clamp 94b, opens the waste liquid clamp 94c, and stops rotation of the main-mechanism-side pump 92 and the upstream pump 142 as illustrated in Fig. 8. With the rotation of the introduction pump 144, the combined sample in the temporary storage unit 136 flows out to the downstream line 138 of the sample introduction channel 130 at a flow rate of, for example, 10 mL/min. As a result, the combined sample flows into the connection part 84 (connection point 134) of the sampling channel 64 from the downstream line 138, sequentially flows through the first detection unit 76 and the second detection unit 80, and is discharged to the waste liquid storage unit 74.

When the combined sample passes, the plurality of first elements 78 (PH chip 78a, O₂ chip 78b, and CO₂ chip 78c) of the first detection unit 76 comes into contact with the combined sample and is colored according to the PH and the contents of O₂ and CO₂. The first measuring instrument 110 optically measures each of the first elements 78, and transmits the detection result to the controller 68. The controller 68 that has received the detection result performs appropriate processing to display the measured values (PH value, O₂ concentration, and CO₂ concentration) on the monitor 100 of the main mechanism 90.

Similarly, when the combined sample passes, the plurality of second elements 82 (glucose chip 82a and lactic acid chip 82b) of the second detection unit 80 comes into contact with the combined sample, and the second measuring instrument 120 detects current values corresponding to the contents of glucose and lactic acid. The second measuring instrument 120 transmits each detection result to the controller 68. The controller 68 that has received the detection result performs appropriate processing to display the measured values (glucose concentration and lactic acid concentration) on the monitor 100.

After the sampling step, the controller 68 determines whether or not the cell culture of the culturing device 11 is completed (step S4 in Fig. 5). When the cell culture is not completed (step S4: NO), the cleaning step (step S5 in Fig. 5) is performed. In the cleaning step, the controller 68 supplies the cleaning solution in the cleaning solution storage unit 70 to the sampling channel 64 to remove the combined sample attached to the plurality of first elements 78 and the plurality of second elements 82.

In addition, the sampling device 60 performs a calibration step (step S6 in Fig. 5) as necessary. In the calibration step, the controller 68 supplies the standard solution in the standard solution storage unit 72 to the sampling channel 64 to calibrate the second sensor unit 121 (second measuring instrument 120). In addition, the user sets the first measuring instrument 110 in the calibration device 118 (see Fig. 1) to calibrate the first measuring instrument 110.

When the cleaning step (or the calibration step) is completed, the controller 68 returns to step S3 and sequentially performs the subsequent steps. On the other hand, when determining in step S4 that the cell culture is completed (step S4: YES), the controller 68 ends the operation flow of the sampling device 60.

Note that the sampling device 60 is not limited to the above configuration, and various methods can be adopted. Some other modifications of the sampling device 60 will be described below.

A sampling device 60A according to a first modification is configured to store the sample from each of the reactors 12 in the temporary storage unit 136 without acquiring information regarding the opening of each of the supply clamps 29A to 29E from the culturing device 11. Note that the configuration of the sampling device 60A may be the same as the configuration of the sampling device 60 described above.

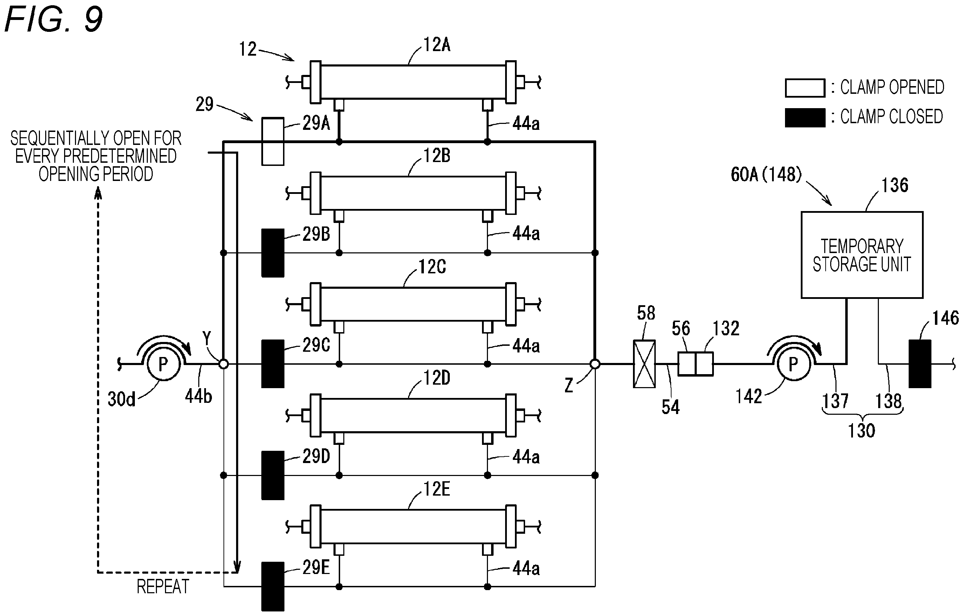

Specifically, the culturing device 11 sequentially performs an operation of opening any one of the supply clamps 29A to 29E and closing the other four in the order of the reactors 12A to 12E for the same period while rotating the EC supply pump 30d as illustrated in Fig. 9. Thus, the culturing device 11 can sequentially and intermittently supply the culture medium to the EC circulation circuit 44a of each of the reactors 12A to 12E. In addition, the culturing device 11 sequentially performs an operation of opening any one of the supply clamps of the IC supply circuits 42b and closing the other four for the same period in the order of the reactors 12A to 12E while rotating the IC supply pump 30b. Thus, the culturing device 11 can sequentially and intermittently supply the culture medium to the EC circulation circuit 44a of each of the reactors 12A to 12E.

On the other hand, the controller 68 of the sampling device 60 operates the upstream pump 142 only for a period (hereinafter referred to as one cycle period) obtained by adding the opening periods of all the supply clamps 29A to 29E. For example, when the opening time of each of the supply clamps 29A to 29E of the culturing device 11 is 6 seconds, the culture medium is supplied from the EC supply circuit 44b to each of the reactors 12A to 12E (EC circulation circuits 44a) once in 30 seconds. The controller 68 sets the time (30 seconds) of one cycle in which each of the supply clamps 29A to 29E is opened once as one cycle period. Then, in a culturing step for supplying and circulating the culture medium of the culturing device 11, the controller 68 rotates the upstream pump 142 for one cycle period at an appropriate timing at which sampling of the culture medium is considered to be necessary. The flow rate of each sample due to the rotation of the upstream pump 142 is set to a value at which a combined sample in a target storage amount can be stored in the temporary storage unit 136. As an example, in a case where one cycle period is 30 seconds and 5 mL of the combined sample is to be stored in the temporary storage unit 136, the upstream pump 142 is rotated so that the flow rate is 10 mL/min.

Here, the sampling device 60 can collect the same amount of samples from the reactors 12A to 12E by operating the upstream pump 142 for one cycle period, even if the rotation start timing of the upstream pump 142 does not coincide with the opening timing of each of the supply clamps 29A to 29E. For example, even if the rotation start timing of the upstream pump 142 is later than the opening timing of the supply clamp 29A by 3 seconds, the supply clamp 29A is opened in the latter half of one cycle period, so that the samples can be stored in the temporary storage unit 136 only during the opening period in which each of the supply clamps 29A to 29E is opened once as a whole.

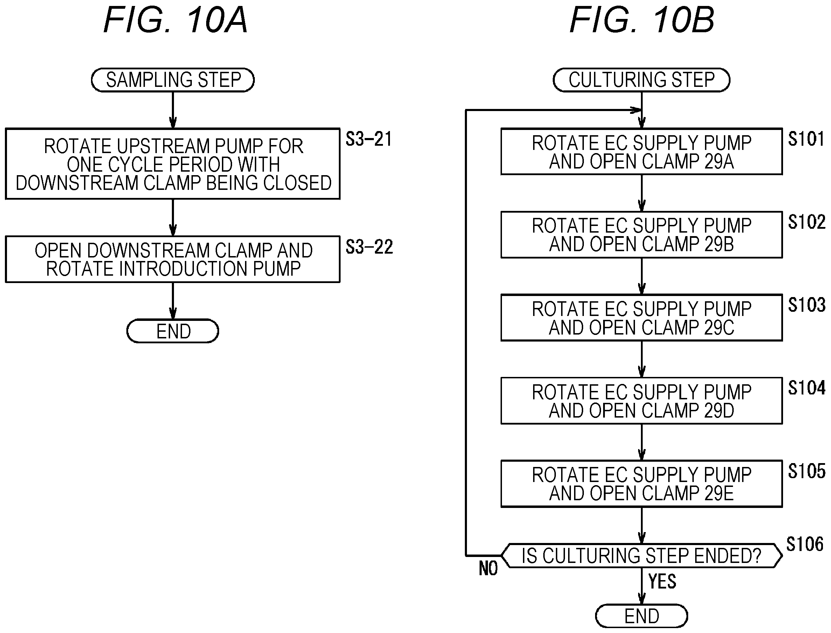

The sampling device 60A according to the first modification is basically configured as described above. The sampling device 60A performs a processing flow illustrated in Fig. 10A in the sampling step (step S3 in Fig. 5). That is, when starting the temporary storage step, the controller 68 rotates the upstream pump 142 for one cycle period while closing the downstream clamp 146 (step S3-21). Note that the sampling device 60A is not limited to rotate the upstream pump 142 only for one cycle period, and may continuously or intermittently execute one cycle period twice or more (a times or more: a is a natural number). Thus, the sampling device 60A can store a sufficient amount of the combined sample in the temporary storage unit 136 even when the supply rate of the culture medium to each of the reactors 12A to 12E is low, for example.

As illustrated in Fig. 10B, during the temporary storage step, the culturing device 11 (control circuit 32) opens the supply clamp 29A for a predetermined opening period while rotating the EC supply pump 30d, and closes the other supply clamps 29B to 29E for the same period as the opening period (step S101). With this process, the culture medium is supplied to the EC circulation circuit 44a of the reactor 12A. Similarly, the culturing device 11 opens the supply clamp 29B for a predetermined opening period while rotating the EC supply pump 30d, and closes the other supply clamps 29A and 29C to 29E for the same period as the opening period (step S102). In addition, the culturing device 11 opens the supply clamp 29C for a predetermined opening period while rotating the EC supply pump 30d, and closes the other supply clamps 29A, 29B, 29D, and 29E for the same period as the opening period (step S103). In addition, the culturing device 11 opens the supply clamp 29D for a predetermined opening period while rotating the EC supply pump 30d, and closes the other supply clamps 29A to 29C and 29E for the same period as the opening period (step S104). In addition, the culturing device 11 opens the supply clamp 29E for a predetermined opening period while rotating the EC supply pump 30d, and closes the other supply clamps 29A to 29D for the same period as the opening period (step S105). With this process, the culture medium is sequentially supplied to the EC circulation circuits 44a of the reactors 12B to 12E. Finally, the culturing device 11 determines whether or not the culturing step is ended (step S106), and when continuing the culturing step (step S106: NO), returns to step S101 and repeats the same flow. When determining that the culturing step is ended (step S106: YES), the culturing device 11 performs the next step (collection step of collecting cells in each of the reactors 12A to 12E).

As illustrated in Fig. 9, the sampling device 60A stores the same amount of samples of the reactors 12A to 12E into the temporary storage unit 136 from the culturing device 11 by rotating the upstream pump 142 for one cycle period in which the culture medium is supplied to each of the reactors 12A to 12E. That is, a combined sample in which the samples are averaged is stored in the temporary storage unit 136.

After the temporary storage step, the controller 68 opens the downstream clamp 146 and rotates the introduction pump 144 (step S3-22). The controller 68 closes the cleaning solution clamp 94a and the standard solution clamp 94b, opens the waste liquid clamp 94c, and stops rotation of the main-mechanism-side pump 92 and the upstream pump 142. As a result, the combined sample in the temporary storage unit 136 flows into the downstream line 138 of the sample introduction channel 130, sequentially flows through the connection part 84 of the sampling channel 64, the first detection unit 76, and the second detection unit 80, and is discharged to the waste liquid storage unit 74.

As described above, by operating the upstream pump 142 for one cycle period, the sampling device 60A can store the combined sample in which the samples are averaged into the temporary storage unit 136 without acquiring the information regarding the opening of each of the supply clamps 29A to 29E from the culturing device 11. In particular, the opening period of the supply clamps 29A to 29E by the culturing device 11 basically does not change. Therefore, periodic sampling can be performed a plurality of times during the culturing step by the culturing device 11 by initially setting one cycle period by the controller 68 of the sampling device 60.

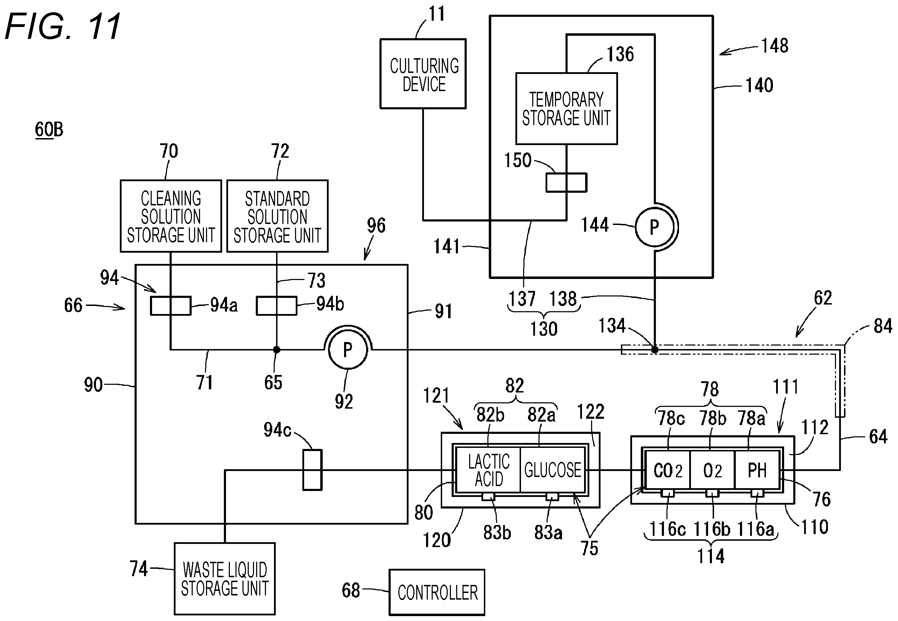

A sampling device 60B according to a second modification is different from the sampling devices 60 and 60A in having an upstream clamp 150 in the upstream line 137 without having the upstream pump 142 and the downstream clamp 146 as illustrated in Fig. 11. Note that, similar to the sampling devices 60 and 60A described above, the introduction pump 144 is provided on the downstream line 138.

In addition, a flexible medical bag that expands due to inflow of the sample and deflates (is flattened) due to outflow of the sample is applied to the temporary storage unit 136. One end of the upstream line 137 is connected to the lower side of the temporary storage unit 136 held by the main unit 96 or the introduction unit 148 in the direction of gravity. In contrast, one end of the downstream line 138 is connected to the upper side of the temporary storage unit 136 held by the main unit 96 or the introduction unit 148 in the direction of gravity.

The sampling device 60B configured as described above rotates the introduction pump 144 while opening the upstream clamp 150 by the controller 68, thereby applying a negative pressure to the upstream line 137 in the temporary storage step. That is, similar to the sampling device 60A, the controller 68 rotates the introduction pump 144 for one cycle period to allow the sample in each of the reactors 12A to 12E to flow into the temporary storage unit 136, and thus, can store the combined sample.

In the combined sample outflow step, the sampling device 60B applies a negative pressure to the temporary storage unit 136 and the downstream line 138 by rotating the introduction pump 144 with the upstream clamp 150 being closed. As a result, the combined sample in the temporary storage unit 136 sequentially flows through the connection part 84 of the sampling channel 64, the first detection unit 76, and the second detection unit 80, and is discharged to the waste liquid storage unit 74.

As described above, the sampling device 60B can store the sample in each of the reactors 12A to 12E into the temporary storage unit 136 under the operation of the introduction pump 144 even when the upstream pump 142 is not provided. Therefore, the configuration of the introduction unit 148 is further simplified to achieve cost reduction, and the introduction unit 148 can be more easily handled.

The sampling device 60B can also allow the sample of the culturing device 11 to flow into the temporary storage unit 136 without relying on the operation of the introduction pump 144. Specifically, the culturing device 11 closes the clamps 28 provided in the IC waste liquid circuit 46 and the EC waste liquid circuit 48, respectively (see Fig. 2). Thus, the culture medium (waste liquid) in each of the reactors 12A to 12E flows out from the EC circulation circuit 44a to the sample introduction channel 130. That is, the sampling device 60B can store the combined sample into the temporary storage unit 136 only by opening the upstream clamp 150 with the introduction pump 144 being stopped. At this time, since the flow rate of the sample flowing out to the sample introduction channel 130 depends on the rotation speed of the EC supply pump 30d, the sampling device 60B only needs to appropriately control the opening period of the upstream clamp 150 according to the rotation speed of the EC supply pump 30d.

Technical ideas and effects that can be grasped from the above embodiment will be described below.

A first aspect of the present invention provides a sampling device 60, 60A, 60B for collecting a sample in a liquid form from a culturing device 11 that has a plurality of reactors 12 for culturing a cell on the basis of a flow of a culture medium, the sampling device including: a sampling channel 64 through which the sample flows; a detection unit 75 provided in the sampling channel 64; a sample introduction channel 130 that connects the sampling channel 64 on an upstream side with respect to the detection unit 75 and the culturing device 11; a pump (upstream pump 142, introduction pump 144) that allows the sample to flow through the sample introduction channel 130; and a control unit (controller 68) that operates the pump, the sampling device including a temporary storage unit 136 provided in the sample introduction channel 130 and capable of temporarily storing the sample, wherein the temporary storage unit 136 sequentially receives flow of the sample for each of the plurality of reactors 12 under the operation of the pump to store a combined sample obtained by combining a plurality of the samples, and sends the combined sample to the sampling channel 64.

With this configuration, the sampling devices 60, 60A, and 60B collectively store the samples in the plurality of reactors 12 into the temporary storage unit 136, and send the combined sample to the sampling channel 64, so that the sample in each of the plurality of reactors 12 can be more efficiently detected. As a result, the sampling devices 60, 60A, and 60B can satisfactorily monitor the culture state of the entire culturing device 11 (all of the plurality of reactors 12) while reducing the number of times of sampling and the sample amount.

In addition, the culturing device 11 includes a plurality of supply channels (EC supply circuits 44b) for supplying a culture medium, the plurality of supply channels being respectively connected to the plurality of reactors 12, and a plurality of supply clamps 29 for opening and closing the plurality of supply channels, respectively, and the control unit (controller 68) operates the pump (upstream pump 142, introduction pump 144) for a predetermined period on the basis of acquisition of information regarding opening of any one of the plurality of supply clamps 29 from the culturing device 11. With this configuration, the sampling device 60 can store the same amount of samples from the plurality of reactors 12 into the temporary storage unit 136, and can more reliably obtain a combined sample in which the samples are averaged.

In addition, the culturing device 11 includes a plurality of supply channels (EC supply circuits 44b) for supplying a culture medium, the plurality of supply channels being respectively connected to the plurality of reactors 12, and a plurality of supply clamps 29 for opening and closing the plurality of supply channels, respectively, and the control unit (controller 68) operates the pump (upstream pump 142, introduction pump 144) for one cycle period obtained by adding opening periods of all of the plurality of supply clamps 29. With this configuration, the sampling device 60A can easily obtain a combined sample in which the samples are averaged without acquiring information regarding opening of the supply clamp 29 from the culturing device 11.

Further, the control unit (controller 68) performs one cycle period for operating the pump (upstream pump 142, introduction pump 144) twice or more. With this configuration, the sampling device 60A can store a sufficient amount of the combined sample in the temporary storage unit 136.

In addition, the pump is an upstream pump 142 provided in the sample introduction channel 130 between the culturing device 11 and the temporary storage unit 136, and the control unit (controller 68) operates the upstream pump 142 in a temporary storage step for allowing the sample in each of the plurality of reactors 12 to flow into the temporary storage unit 136, and stops the operation of the upstream pump 142 in a combined sample outflow step for sending the combined sample from the temporary storage unit 136 to the sampling channel 64. With this configuration, the sampling device 60 can stably store the sample in each of the plurality of reactors 12 into the temporary storage unit 136 in the temporary storage step.

In addition, the sample introduction channel 130 includes a downstream clamp 146 that opens and closes the sample introduction channel 130 between the sampling channel 64 and the temporary storage unit 136, and the control unit (controller 68) closes the downstream clamp 146 in the temporary storage step and opens the downstream clamp 146 in the combined sample outflow step. With this configuration, the sampling devices 60 and 60A can prevent the sample from flowing out of the temporary storage unit 136 in the temporary storage step.

In addition, the sample introduction channel 130 has an introduction pump 144 between the sampling channel 64 and the temporary storage unit 136, and the control unit (controller 68) stops operation of the introduction pump 144 in the temporary storage step, and operates the introduction pump 144 in the combined sample outflow step to send the combined sample to the sampling channel 64. With this configuration, the sampling devices 60 and 60A can smoothly introduce the combined sample into the sampling channel 64 in the combined sample outflow step.

In addition, the pump is an introduction pump 144 provided in the sample introduction channel 130 between the sampling channel 64 and the temporary storage unit 136, and the control unit (controller 68) operates the introduction pump 144 in a temporary storage step for allowing the sample in each of the plurality of reactors 12 to flow into the temporary storage unit 136, and stops operation of the introduction pump 144 in a combined sample outflow step for sending the combined sample from the temporary storage unit 136 to the sampling channel 64. With this configuration, the sampling device 60B can guide the sample in each of the plurality of reactors 12 to the temporary storage unit 136 by applying a negative pressure to the sample introduction channel 130 on the upstream side of the introduction pump 144 under the operation of the introduction pump 144 in the temporary storage step. Furthermore, the sampling device 60B can achieve cost reduction by eliminating the upstream pump 142.

In addition, the sample introduction channel 130 includes an upstream clamp 150 that opens and closes the sample introduction channel 130 between the culturing device 11 and the temporary storage unit 136, and the control unit (controller 68) opens the upstream clamp 150 in the temporary storage step and closes the upstream clamp 150 in the combined sample outflow step. With this configuration, the sampling device 60B can store the sample in each of the plurality of reactors 12 into the temporary storage unit 136 in the temporary storage step, and introduce the combined sample to the sampling channel 64 in the combined sample outflow step.

A second aspect of the present invention provides a cell culturing system 10 for collecting a sample in a liquid form from a culturing unit (culturing device 11) that has a plurality of reactors 12 for culturing a cell on the basis of a flow of a culture medium, the cell culturing unit sequentially supplying the culture medium to the plurality of reactors 12, the cell culturing system including: a sampling channel 64 through which the sample flows; a detection unit 75 provided in the sampling channel 64; a sample introduction channel 130 that connects the sampling channel 64 on an upstream side with respect to the detection unit 75 and the culturing unit; a pump (upstream pump 142, introduction pump 144) that allows the sample to flow through the sample introduction channel 130; and a control unit (controller 68) that operates the pump, the cell culturing system including a temporary storage unit 136 provided in the sample introduction channel 130 and capable of temporarily storing the sample, wherein the temporary storage unit 136 sequentially receives flow of the sample for each of the plurality of reactors 12 under the operation of the pump to store a combined sample obtained by combining a plurality of the samples, and sends the combined sample to the sampling channel 64. With this configuration, the cell culturing system 10 can more efficiently detect a sample for each of the plurality of reactors 12.
FIG. 2
   - 14: CULTURE MEDIUM RESERVOIR
   - 20: WASTE LIQUID UNIT
   - 32: CONTROL CIRCUIT
FIG. 3
   - 136: TEMPORARY STORAGE UNIT
FIG. 4
   - 11: CULTURING DEVICE
   - 70: CLEANING SOLUTION STORAGE UNIT
   - 72: STANDARD SOLUTION STORAGE UNIT
   - 74: WASTE LIQUID STORAGE UNIT
GLUCOSE
LACTIC ACID
   - 68: CONTROLLER
   - 136: TEMPORARY STORAGE UNIT
FIG. 5
   - S1: PREPARATION STEP
   - S2: PRIMING STEP
   - S3: SAMPLING STEP
   - S4: HAS CELL CULTURE BEEN COMPLETED?
   - S5: CLEANING STEP
   - S6: CALIBRATION STEP
FIG. 6
SAMPLING STEP
S3-1 ACQUIRE INFORMATION REGARDING ROTATION OF EC SUPPLY PUMP AND OPENING OF CLAMP 29A FROM CULTURING DEVICE
S3-2 ROTATE UPSTREAM PUMP FOR PREDETERMINED PERIOD WHILE CLOSING DOWNSTREAM CLAMP
S3-3 ACQUIRE INFORMATION REGARDING ROTATION OF EC SUPPLY PUMP AND OPENING OF CLAMP 29B FROM CULTURING DEVICE
S3-4 ROTATE UPSTREAM PUMP FOR PREDETERMINED PERIOD WHILE CLOSING DOWNSTREAM CLAMP
S3-5 ACQUIRE INFORMATION REGARDING ROTATION OF EC SUPPLY PUMP AND OPENING OF CLAMP 29C FROM CULTURING DEVICE
S3-6 ROTATE UPSTREAM PUMP FOR PREDETERMINED PERIOD WHILE CLOSING DOWNSTREAM CLAMP
S3-7 ACQUIRE INFORMATION REGARDING ROTATION OF EC SUPPLY PUMP AND OPENING OF CLAMP 29D FROM CULTURING DEVICE
S3-8 ROTATE UPSTREAM PUMP FOR PREDETERMINED PERIOD WHILE CLOSING DOWNSTREAM CLAMP
S3-9 ACQUIRE INFORMATION REGARDING ROTATION OF EC SUPPLY PUMP AND OPENING OF CLAMP 29E FROM CULTURING DEVICE
S3-10 ROTATE UPSTREAM PUMP FOR PREDETERMINED PERIOD WHILE CLOSING DOWNSTREAM CLAMP
S3-11 OPEN DOWNSTREAM CLAMP AND ROTATE INTRODUCTION PUMP TEMPORARY STORAGE STEP
COMBINED SAMPLE OUTFLOW STEP
FIG. 7
   - 11: CULTURING DEVICE
   - 70: CLEANING SOLUTION STORAGE UNIT
   - 72: STANDARD SOLUTION STORAGE UNIT
   - 74: WASTE LIQUID STORAGE UNIT
   - 68: CONTROLLER
GLUCOSE
LACTIC ACID
   - 136: TEMPORARY STORAGE UNIT
CLAMP OPENED
CLAMP CLOSED
FIG. 8
   - 11: CULTURING DEVICE
   - 70: CLEANING SOLUTION STORAGE UNIT
   - 72: STANDARD SOLUTION STORAGE UNIT
   - 74: WASTE LIQUID STORAGE UNIT
   - 68: CONTROLLER
GLUCOSE
LACTIC ACID
   - 136: TEMPORARY STORAGE UNIT
CLAMP OPENED
CLAMP CLOSED
FIG. 9
SEQUENTIALLY OPEN FOR EVERY PREDETERMINED OPENING PERIOD REPEAT
CLAMP OPENED
CLAMP CLOSED
136 TEMPORARY STORAGE UNIT
FIG. 10A
SAMPLING STEP
S3-21 ROTATE UPSTREAM PUMP FOR ONE CYCLE PERIOD WITH DOWNSTREAM CLAMP BEING CLOSED
S3-22 OPEN DOWNSTREAM CLAMP AND ROTATE INTRODUCTION PUMP
FIG. 10B
CULTURING STEP
   - S101: ROTATE EC SUPPLY PUMP AND OPEN CLAMP 29A
   - S102: ROTATE EC SUPPLY PUMP AND OPEN CLAMP 29B
   - S103: ROTATE EC SUPPLY PUMP AND OPEN CLAMP 29C
   - S104: ROTATE EC SUPPLY PUMP AND OPEN CLAMP 29D
   - S105: ROTATE EC SUPPLY PUMP AND OPEN CLAMP 29E
   - S106: IS CULTURING STEP ENDED?
FIG. 11
   - 11: CULTURING DEVICE

   - 70: CLEANING SOLUTION STORAGE UNIT
   - 72: STANDARD SOLUTION STORAGE UNIT
   - 74: WASTE LIQUID STORAGE UNIT
   - 68: CONTROLLER
GLUCOSE
LACTIC ACID
   - 136: TEMPORARY STORAGE UNIT

## Claims

1. A sampling device for collecting a sample in a liquid form from a culturing device that has a plurality of reactors for culturing a cell on the basis of a flow of a culture medium, the sampling device comprising:
a sampling channel through which the sample flows;
a detection unit provided in the sampling channel;
a sample introduction channel that connects the sampling channel on an upstream side with respect to the detection unit and the culturing device;
a pump that allows the sample to flow through the sample introduction channel; and
a control unit that operates the pump,
the sampling device including
a temporary storage unit provided in the sample introduction channel and capable of temporarily storing the sample, wherein
the temporary storage unit sequentially receives flow of the sample for each of the plurality of reactors under the operation of the pump to store a combined sample obtained by combining a plurality of the samples, and sends the combined sample to the sampling channel.

2. The sampling device according to claim 1, wherein
the culturing device includes a plurality of supply channels for supplying a culture medium, the plurality of supply channels being respectively connected to the plurality of reactors, and a plurality of supply clamps for opening and closing the plurality of supply channels, respectively, and
the control unit operates the pump for a predetermined period on the basis of acquisition of information regarding opening of any one of the plurality of supply clamps from the culturing device.

3. The sampling device according to claim 1, wherein
the culturing device includes a plurality of supply channels for supplying a culture medium, the plurality of supply channels being respectively connected to the plurality of reactors, and a plurality of supply clamps for opening and closing the plurality of supply channels, respectively, and
the control unit operates the pump for one cycle period obtained by adding opening periods of all of the plurality of supply clamps.

4. The sampling device according to claim 3, wherein
the control unit performs the one cycle period for operating the pump twice or more.

5. The sampling device according to any one of claims 1 to 4, wherein
the pump is an upstream pump provided in the sample introduction channel between the culturing device and the temporary storage unit, and
the control unit operates the upstream pump in a temporary storage step for allowing the sample in each of the plurality of reactors to flow into the temporary storage unit, and stops the operation of the upstream pump in a combined sample outflow step for sending the combined sample from the temporary storage unit to the sampling channel.

6. The sampling device according to claim 5, wherein
the sample introduction channel includes a downstream clamp that opens and closes the sample introduction channel between the sampling channel and the temporary storage unit, and
the control unit closes the downstream clamp in the temporary storage step and opens the downstream clamp in the combined sample outflow step.

7. The sampling device according to claim 5 or 6, wherein
the sample introduction channel has an introduction pump between the sampling channel and the temporary storage unit, and
the control unit stops operation of the introduction pump in the temporary storage step, and operates the introduction pump in the combined sample outflow step to send the combined sample to the sampling channel.

8. The sampling device according to any one of claims 1 to 4, wherein
the pump is an introduction pump provided in the sample introduction channel between the sampling channel and the temporary storage unit, and
the control unit operates the introduction pump in a temporary storage step for allowing the sample in each of the plurality of reactors to flow into the temporary storage unit, and stops the operation of the introduction pump in a combined sample outflow step for sending the combined sample from the temporary storage unit to the sampling channel.

9. The sampling device according to claim 8, wherein
the sample introduction channel includes an upstream clamp that opens and closes the sample introduction channel between the culturing device and the temporary storage unit, and
the control unit opens the upstream clamp in the temporary storage step and closes the upstream clamp in the combined sample outflow step.

10. A cell culturing system for collecting a sample in a liquid form from a culturing unit that has a plurality of reactors for culturing a cell on the basis of a flow of a culture medium,
the cell culturing unit sequentially supplying the culture medium to the plurality of reactors,
the cell culturing system comprising:
a sampling channel through which the sample flows;
a detection unit provided in the sampling channel;
a sample introduction channel that connects the sampling channel on an upstream side with respect to the detection unit and the culturing unit;
a pump that allows the sample to flow through the sample introduction channel; and
a control unit that operates the pump,
the cell culturing system including
a temporary storage unit provided in the sample introduction channel and capable of temporarily storing the sample, wherein
the temporary storage unit sequentially receives flow of the sample for each of the plurality of reactors under the operation of the pump to store a combined sample obtained by combining a plurality of the samples, and sends the combined sample to the sampling channel.
